# FASCICULE DE BREVET EUROPEEN

(11) **EP 0 378 483 B1**
(45) Date de publication et mention de la délivrance du brevet: **14.08.1996**
(21) Numéro de dépôt: 90400081.7
(22) Date de dépôt: 11.01.1990
(51) Int. Cl.: A61K 31/505

(54) **Utilisation de la 4-mercapto 6-hydroxy pyrazolo(4,3-d)pyrimidine comme capteur de radicaux libres**
Verwendung von 4-Mercapto-6-hydroxy-pyrazolo(4,3-d)-pyrimidine als Radikalfänger
Use of 4-mercapto-6-hydroxy-pyrazolo-(4,3-d)-pyrimidine as a scavenger of free radicals

(30) Priorité: 11.01.1989 FR 8900353
(43) Date de publication de la demande: 18.07.1990
(73) Titulaire: BOUCHARA S.A., F-92300 Levallois-Perret (FR)
(72) Inventeur: Pierce, Belinda, Uxbridge,Middelesex UB 8 3PH (GB)
(74) Mandataire: Burtin, Jean-François Bouchara S.A.

(56) Documents cités:
- EP-A- 0 237 348
- EP-A- 0 274 928
- US-A- 3 474 098
- US-A- 3 519 716
- C.R. ACAD. SC. PARIS, vol. 284, série D, 7 février 1977, pages 481-483; K.V. THANG et al.: "Inactivation, par des pyrazolo [3,4-d] pyrimidines 4.6 disubstituées, de l'arsénolyse de la guanosine par la purine nucléoside phosphorylase érythrocytaire"
- ISRAEL JOURNAL OF CHEMISTRY, vol. 6, 1963, pages 787-796; G.B. ELION et al.: "Effects of xanthine oxidase inhibitors on purine catabolism"

## Description

La présente invention concerne l'emploi en thérapeutique d'une pyrazolopyrimidine connue.

Elle a plus particulièrement pour objet l'emploi comme capteur (Scavenger) de radicaux libres d'une pyrazolopyrinidine et son emploi pour le traitement des séquelles d'hypoxie ou d'anoxie tissulaire.

Elle concerne spécifiquement l'emploi comme capteur de radicaux libres de la 4-mercapto 6-hydroxy pyrazolo (4,3-d) pyrimidine et de ses sels d'addition avec un acide minéral ou organique.

Ce composé déjà décrit dans la littérature est considéré comme le métabolite principal du Thiopurinol (4-mercapto pyrazolo (4,3-d) pyrimidine. Il est généralement préparé par oxydation enzymatique du Thiopurinol au moyen de Xanthine oxydase du lait fixée sur Sepharose par liaison à l'aide du bromure de Cyanogène (cf. Pasquier et C. Auscher Europ. J. of Drug Metabolism and Pharm. 1979 p. 59). Les propriétés de composé synthétique ainsi obtenu, sont identiques à celles du produit isolé de l'urine des sujets traités par le Thiopurinol.

Aucune propriété thérapeutique n'était connue jusqu'ici pour la 4-mercapto 6 hydroxypyrazolo (3,4-d) pyrimidine.

Le rôle des radicaux libres dans les lésions cardiaques, digestives, pancréatiques et pulmonaires provoquées par la reperfusion a été étudié expérimentalement, mais, plus récemment, on a également tenté d'établir une corrélation entre les radicaux libres et les lésions cérébrales secondaires à une ischémie-reperfusion.

Normalement, l'oxygène est métabolisé en deux molécules d'eau par l'addition de quatre électrons liés à quatre atomes d'hydrogène. Ce processus se déroule dans les mitochondries sous le contrôle enzymatique de la cytochromeoxydase.

Lors de chaque adjonction d'un électron à une molécule d'oxygène, des composés hautement réactifs sont produits en présence d'ions hydrogène. Un électron forme un radical anionique superoxyde, un deuxième, un peroxyde d'hydrogène, et un troisième, le radical hydroxyl.

Habituellement, la cytochrome oxydase empêche les radicaux intermédiaires de réagir avec les autres molécules voisines, ou bien les radicaux sont éliminés par des enzymes destructrices, telles que superoxyde dismutase, catalase, ou glutathion peroxydase.

En cas d'ischémie tissulaire, les composants cellulaires normaux se dégradent. Ces produits de dégradation constituent alors le substrat de réactions aboutissant à la production de radicaux libres, lors de l'apport d'oxygène secondaire à la réperfusion. Pendant l'ischémie, l'hypoxanthine, par exemple, s'accumule par dégradation de l'adénosine triphosphate et une accumulation d'acides gras se produit par dégradation des phospholipides.

Certaines enzymes sont également altérées au cours de l'ischémie et catalysent alors des réactions qui produisent des radicaux comme produits terminaux. La xanthine déshydrogénase, par exemple, catalyse habituellement une réaction dont le produit terminal est l'acide urique. Le produit ischémique convertit la xanthine déshydrogénase en xanthine oxydase, qui catalyse la production de radicaux à partir de l'oxygène et de l'hypoxanthine.

Il semble démontré que les radicaux sont impliqués dans les lésions cérébrales provoquées par l'ischémie-reperfusion. Le cerveau est particulièrement exposé à ces lésions car il contient des taux élevés d'acides gras peroxydables et des taux relativement faibles d'enzymes protectrices (R. Floyd).

Floyd et ses collègues ont utilisé des techniques électrochimiques pour détecter la réaction chimique impliquant les radicaux libres hydroxyl dans le cerveau de la gerbille.

Ils ont montré que l'ischémie ne suffisait pas, à elle seule, à provoquer la réaction chimique et les lésions tissulaires, mais que la reperfusion était nécessaire.

Le mécanisme des lésions de reperfusion se met en place pendant l'ischémie, avec formation de xanthine oxydase et de son substrat, l'hypoxanthine, un produit de dégradation de l'adénosine triphosphate. Lorsque le débit sanguin est rétabli dans les tissus, la réintroduction de l'oxygène moléculaire déclenche la formation de superoxydes.

Kontos a expliqué les mécanismes possibles des lésions dues aux radicaux oxygène. D'après lui, une fois produits, les radicaux oxygène réagissent avec les acides gras insaturés des membranes cellulaires, des protéines, des enzymes et de l'ADN. Au niveau du cerveau, par exemple, les cellules nerveuses sont lésées, ce qui altère l'activité électrique cérébrale.

Floyd a démontré que la synthèse des neurotransmetteurs, en particulier de la dopamine, est affectée par la peroxydation du tissu nerveux. Selon lui, les lésions des membranes neuronales ou des enzymes catalysant la réaction en sont responsables.

Kontos a également émis l'hypothèse que les cellules endothéliales vasculaires et musculaires lisses sont endommagées, conduisant à une dilatation des artérioles cérébrales et à une effraction de la barrière hémoméningée. Il admet cependant qu'il est parfois difficile de déterminer quelle est la part de responsabilité respective, dans les lésions observées, de l'ischémie et des radicaux.

On a déjà pourtant décrit de nombreuses substances pouvant permettre de détruire les radicaux oxygène pendant la reperfusion. La vitamine E a été proposée, mais le malade doit avoir un taux sanguin élevé de vitamine E avant la survenue de l'ischémie, ce qui est difficile à obtenir dans la mesure où celle-ci est inattendue. Sa concentration au niveau des lésions doit également être élevée, ce qui nécessite l'administration d'une quantité importante et parfois intolérable. La vitamine E se révèle donc inutilisable en pratique.

La superoxyde dismutase a été utilisée avec succès chez le chat pour corriger les modifications vasculaires ischémiques. A la différence de la vitamine E, elle peut être administrée juste avant la reperfusion.

Une autre approche consiste à diminuer la production des radicaux. L'allopurinol inhibe la xanthine oxydase, mais présente les mêmes contraintes que la vitamine E. Ginsberg pense que l'allopurinol peut être plus utile dans les tissus possédant des taux enzymatiques élevés, tels que le coeur.

Les chélateurs, tels que l'acide édétique et la desferrioxamine, qui se lient aux atomes de fer des enzymes, représentent d'autres alternatives possibles.

La 4-mercapto 6-hydroxy pyrazolo (4,3-d) pyrimidine trouve une application en thérapeutique en tant qu'inhibiteur de la synthèse de l'acide urique et en tant qu'anti-oxydant. En tant que tel, on l'utilise dans les systèmes ischémie-reperfusion d'organes et elle protège les tissus contre les lésions provoquées par les radicaux libres.

Les radicaux libres, en effet, apparaissent dans des tissus privés d'oxygène à la suite d'une ischémie suivie d'une reperfusion au cours de laquelle a lieu un apport important d'oxygène ; leurs origines sont nombreuses, les deux sources principales sont :
1) la xanthine oxydase produisant des anions superoxydes lorsqu'elle catalyse l'oxydation de la xanthine ou de l'hypoxanthine en acide urique ;
2) Les polynucléaires neutrophiles qui, lorsqu'ils sont stimulés, produisent des anions superoxydes.

La présence d'anions superoxydes entraîne la formation de dérivés tels que le peroxyde d'hydrogène, radical très réactifs, et des peroxydes lipidiques pouvant se former à partir des lipides membranaires.

D'après les résultats obtenus, il semble que Oxythiopurinol soit meilleur protecteur que l'Allopurinol, vis-à-vis des radicaux hydroxyles, ainsi que vis-à-vis des peroxydes.

Les applications cliniques directes prévues peuvent être :
1) Protection de différents organes au cours d'interventions chirurgicales (CEC ou transplantation) ;
2) Anti-inflammatoire : l'inflammation étant une conséquence d'une accumulation de polynucléaires activés ;
3) Radioprotecteur : au cours d'irradiations médicales, afin de protéger certains tissus contre les radio-lésions ;
4) Protection contre la cataracte dont l'étiologie est liée à l'existence de radicaux libres ;
5) Le traitement des rétinopathies et des dégénérescences.
6) Dans le domaine de la pathologie des voies respiratoires
   - soulagement des symptômes inflammatoires aigus
   - prévention des surinfections
   - protection renforcée contre la survenue des rhinites atopiques ou croûteuses (effet-lésion).

A ces fins, la 4-mercapto 6-hydroxy pyrazolo (4,3-d) pyramidine et ses sels est administrée par voie buccale ou parentérale sous forme de compositions pharmaceutiques.

Dans celles-ci, la 4-mercapto 6-hydroxypurazolo (4,3-d) pyrimidine ou un de ses sels est mélangée, ou ajoutée, ou dissoute dans un véhicule ou un excipient inerte, non toxique, pharmaceutiquement acceptable.

On pourra citer, à cet égard, les solutés en suspensions injectables pour perfusion veineuse, les comprimés, les dragées, les gélules, les poudres aromatisées, ou non, présentées en sachets, les comprimés effervescents.

La posologie usuelle varie selon la voie d'administration, l'âge du sujet et l'indication thérapeutique. Elle s'échelonne de 100 à 600 mg par jour.

La 4-mercapto 6-hydroxypyrazolo (4,3-d) pyrimidine ou un de ses sels est présentée de préférence sous forme de préparations destinées à la voie buccale comme des comprimés ou des gélules renfermant 100, 200 ou 300 mg de principe actif.

L'invention s'étend aussi à un procédé d'obtention des compositions pharmaceutiques à base de 4-mercapto 6-hydroxy pyrazolo (4,3-d) pyrimidine ou d'un de ses sels selon l'invention caractérisé en ce que l'on incorpore ou mélange la 4-mercapto 6-hydroxypyrazolo (4,3-d) pyrimidine, ou un de ses sels avec un excipient ou un véhicule non toxique selon les méthodes connues en pharmacotechnie.

## Revendications (Revendications pour l'(les) Etat(s) contractant(s) suivant(s): AT, BE, CH, DE, DK, FR, GB, IT, LI, LU, NL, SE)

1. Utilisation de la 4-mercapto 6-hydroxy pyrazolo (4,3-d) pyrimidine ou d'un de ses sels, en vue de la production d'un médicament dans le domaine de la pathologie des voies respiratoires, notamment pour le soulagement des symptômes inflammatoires aigus, la prévention des surinfections et la protection renforcée contre la survenue des rhinites atopiques ou croûteuses.

2. Utilisation de la 4-mercapto 6-hydroxy pyrazolo (4,3-d) pyrimidine ou d'un de ses sels en vue de la production d'un médicament pour la protection des complications vasculaires liées à l'anoxie ou à l'ischémie.

3. Utilisation selon l'une des revendications 1 ou 2, dans laquelle le principe actif est la 4-mercapto 6-hydroxy pyrazolo (4,3-d) pyrimidine.

4. Utilisation selon l'une des revendications 1 à 3, dans laquelle la teneur en principe actif du médicament s'échelonne de 100 à 300 mg par dose unitaire pour la voie buccale.

5. Procédé d'obtention des compositions pharmaceutiques pour l'utilisation visée à la revendication 1 ou à la revendication 2, caractérisé en ce que l'on mélange ou incorpore la 4-mercapto 6-hydroxy pyrazolo (4,3-d) pyrimidine ou un de ses sels, avec un excipient ou un véhicule inerte, non toxique, pharmaceutiquement acceptable.

## Revendications (Revendications pour l'(les) Etat(s) contractant(s) suivant(s): GR, ES)

1. Un procédé d'obtention de compositions pharmaceutiques caractérisé en ce que le principe actif la 4-mercapto 6-hydroxy pyrazolo (4,3-d ) pyrimidine ou un de ses sels, est mis en association ou en mélange avec un excipient ou un véhicule inerte, non toxique, pharmaceutiquement acceptable en vue de la production d'un médicament dans le domaine de la pathologie des voies respiratoires, notamment pour le soulagement des symptômes inflammatoires aigus, la prévention des surinfections et la protection renforcée contre la survenue des rhinites atopiques ou croûteuses.

2. Un procédé d'obtention de compositions pharmaceutiques caractérisé en ce que le principe actif la 4-mercapto 6-hydroxy pyrazolo (4,3-d ) pyrimidine ou un de ses sels, est mis en association ou en mélange avec un excipient ou un véhicule inerte, non toxique, pharmaceutiquement acceptable en vue de la production d'un médicament pour la protection des complications vasculaires liées à l'anoxie ou à l'ischémie.

3. Un procédé d'obtention de compositions pharmaceutiques selon l' une des revendications 1 ou 2, dans lequel la teneur en principe actif du médicament s'échelonne de 100 à 300 mg par dose unitaire pour la voie buccale.

## Claims (Claims for the following Contracting State(s): AT, BE, CH, DE, DK, FR, GB, IT, LI, LU, NL, SE)

1. Use of 4 -mercapto 6 -hydroxy pyrazolo ( 4,3-d ) pyrimidine or one of its salts, for the production of a drug in the pathology domain of the respiratory ways, especially for the alleviation of acute inflammatory symptoms, the prevention of superinfection and reinforced protection against the occurrence of atopics or crusteous rhinitis.

2. Use of 4 -mercapto 6 -hydroxy pyrazolo ( 4,3-d ) pyrimidine or one of its salts, for the production of a drug for the protection against vascular complications connected to anoxia or ischemia.

3. Use according to any of claims 1 or 2, in which the active ingredient is 4 -mercapto 6 - hydroxy pyrazolo ( 4,3,-d ) pyrimidine.

4. Use according to any of claims 1 to 3, in which the amount of active ingredient of the drug ranges from 100 to 300 mg per unit dosage for the oral way.

5. A process for producing pharmaceutical compositions for the intented use according to claim 1 or to claim 2, wherein the 4 -mercapto 6 -hydroxy pyrazolo ( 4,3-d ) pyrimidine or one of its salts is mixed or incorporated with an inert, non toxic, pharmaceutically-acceptable vehicle or carrier.

## Claims (Claims for the following Contracting State(s): GR, ES)

1. A process for producing pharmaceutical compositions wherein the active ingredient, 4 -mercapto 6 -hydroxy pyrazolo ( 4,3-d ) pyrimidine or one of its salts, is in association or in admixture with an inert, non toxic, pharmaceutically-acceptable vehicle or carrier, for the production of a drug in the pathology domain of the respiratory ways, especially for the alleviation of acute inflammatory symptoms, the prevention of superinfection and reinforced protection against the occurrence of atopics or crusteous rhinitis.

2. A process for producing pharmaceutical compositions wherein the active ingredient 4 -mercapto 6 -hydroxy pyrazolo ( 4,3-d ) pyrimidine or one of its salts, is in association or in admixture with an inert, non toxic, pharmaceutically acceptable vehicle or carrier for the production of a drug for the protection against vascular complications connected to anoxia or ischemia.

3. A process for producing pharmaceutical compositions according to any of claims 1 to 2, wherein the amount of active ingredient of the drug ranges from 100 to 300 mg per unit dosage for the oral way.

## Patentansprüche (Patentansprüche für folgende(n) Vertragsstaat(en): AT, BE, CH, DE, DK, FR, GB, IT, LI, LU, NL, SE)

1. Verwendung von 4-Mercapto 6-Hydroxy Pyrazolo ( 4,3-d ) Pyrimidin, oder eines seiner Salze, für die Herstellung eines Arzneimittels in dem Bereich von der Pathologie des Atemweges, nämlich für die Linderung des akuten Entzündungs symptome für die Verhütung der Zwischeninfektionen und für die verstärkte Schutz gegen den Anfallen des atopischer oder verkruste Rhinitis der Nasenschleimhautentzüdung.

2. Verwendung von 4-Mercapto 6-Hydroxy Pyrazolo ( 4,3,-d ) Pyrimidin, oder eine seiner Salze, für die Herstellung eines Arzneimittels für die Schutz der Blutgefäß gegen die Komplikationen die mit Anoxia oder Ischemia gebunden sind.

3. Verwendung nach einer der Ansprüche 1 oder 2, worin den Wirkstoff das 4-Mercapto 6-Hydroxy Pyrazolo (4,3-d ) Pyrimidin ist.

4. Vewendung nach einer der Ansprüche 1 bis 3, worin der Gehalt an Wirkstoff von 100 bis 300 mg per Verabreichungseinheit für die orale Abreichung liegt.

5. Verfahren zur Herstellung von pharmazeutische Zusammensetzungen für die in Anspruch 1 oder Anspruch 2 gezielte Verwendung, worin das 4-Mercapto 6-Hydroxy Pyrazolo ( 4,3-d ) Pyrimidin, oder eine seiner Salze, mit einem inerten, unbedenklichen, pharmazeutischverträglichen Träger oder Vehikeln, in Verbindung oder Vermischung gesetzt ist.

## Patentansprüche (Patentansprüche für folgende(n) Vertragsstaat(en): GR, ES)

1. Verfahren zur Herstellung von pharmazeutische Zusammensetzungen dadurch gekennzeichnet dass der Wirkstoff, das 4-Mercapto 6-Hydroxy Pyrazolo ( 4,3-d ) Pyrimidin oder eine seiner Salze, mit einem inerten, unbedenklichen, pharmazeutischverträglichen Träger oder Vehikeln, in Verbindung oder Vermischung gesetzt ist, für die Herstellung eines Arzneimittels in dem Bereich von der Pathologie des Atemweges, nämlich für die Linderung der akuten Entzündungssymptome, die Verhütung der Zwischeninfektionen und für die verstärkte Schutz gegen den Anfallen des atopischer oder verkruste Rhinitis der Nasenschleimhautentzündung.

2. Verfahren zur Herstellung von pharmazeutische Zusammensetzungen dadurch gekennzeichnet dass der Wirkstoff, das 4-Mercapto 6-Hydroxy Pyrazolo ( 4,3-d ) Pyrimidin oder eine seiner Salze, mit einem inerten, unbedenklichen, pharmazeutischverträglichen Träger oder Vehikeln, in Verbindung oder Vermischung gesetzt ist, für die Herstellung eines Arzneimittel für die Schutz der Blutgefäß gegen die Komplikationen die mit Anoxia und Ischemia gebunden sind.

3. Verfahren zur Herstellung von pharmazeutische Zusammensetzungen nach einer der Ansprüche 1 oder 2, worin der Gehalt an Wirkstoff in dem Arzneimittel von 100 bis 300 mg per Verabreichungseinheit für die orale Abreichung liegt.
